# EUROPÄISCHE PATENTSCHRIFT

(11) **EP 3 108 903 B1**
(45) Veröffentlichungstag und Bekanntmachung des Hinweises auf die Patenterteilung: **13.03.2024**
(21) Anmeldenummer: 16001397.5
(22) Anmeldetag: 21.06.2016
(51) Int. Cl.: A61L 2/22, A61L 2/24, A61L 9/14

(54) **DESINFEKTIONSEINRICHTUNG ZUM DESINFIZIEREN VON RÄUMEN SOWIE VERFAHREN ZUM DESINFIZIEREN VON RÄUMEN**
DISINFECTION DEVICE FOR DISINFECTING ROOMS AND METHOD FOR DISINFECTING ROOMS
DISPOSITIF DE DESINFECTION DE PIECES ET PROCEDE DE DESINFECTION DE PIECES

(30) Priorität: 25.06.2015 DE 102015008589
(43) Veröffentlichungstag der Anmeldung: 28.12.2016
(73) Patentinhaber: Swingtec GmbH, 88316 Isny (DE)
(72) Erfinder: Dietrich, Volker, 88239 Wangen (DE)
(74) Vertreter: Kohl, Karl-Heinz

(56) Entgegenhaltungen:
- WO-A1-2014/076013
- WO-A2-2012/033850
- AT-U1- 11 061
- AT-U1- 11 061
- GB-A- 2 499 819
- US-A1- 2008 038 166
- US-A1- 2013 302 208
- US-B1- 8 382 008

## Beschreibung

Die Erfindung betrifft eine Desinfektionseinrichtung zum Desinfizieren von Räumen nach dem Oberbegriff des Anspruches 1 sowie ein Verfahren zum Desinfizieren von Räumen nach dem Oberbegriff des Anspruches 11.

Zum Desinfizieren von Räumen, insbesondere von Krankenhausräumen, ist es bekannt, dass in den zu desinfizierenden Raum ein Desinfektionsmittel ausgebracht wird. Das Desinfektionsmittel befindet sich in einem Behälter, an den eine Austragdüse über eine Leitung angeschlossen ist. Das Desinfektionsmittel wird durch das Venturiprinzip aus dem Behälter gefördert, so dass es aus der Austragdüse austritt. Damit eine den gesetzlichen Forderungen entsprechende Desinfizierung erreicht wird, müssen bestimmte Grenzwerte eingehalten werden. So muss die Raumtemperatur sowie die relative Feuchte im Raum oberhalb eines Mindestwertes liegen. Die Konzentration an Desinfektionsmittel im Raum muss während des Desinfektionsvorganges oberhalb eines vorgeschriebenen Mindestwertes liegen. Nach jeder Messung dieser Parameter muss gegebenenfalls der Raum betreten werden, um beispielsweise Wasser in den Raum zu sprühen, wenn die relative Feuchte im Raum unterhalb des Mindestwertes liegt. Auch muss der Raum betreten werden, wenn beispielsweise die Konzentration an Desinfektionsmittel zu gering ist. Das Betreten des zu desinfizierenden Raumes bedeutet, dass der Raum nach Verlassen der jeweiligen Person stets neu abgedichtet werden muss. Ein solches Vorgehen ist sehr zeitaufwändig und umständlich. Beispiele für Systeme zur Desinfektion von Räumen sind in WO 2012/033850, GB 2 499 819, AT 11 061, US 2008/038166, US 8 382 008, US 2013/302208, WO 2014/076013 dargestellt, bei denen das Desinfektionsmittel mittels eines Sprühverfahrens kontrolliert in den Raum eingebracht wird und der Gehalt an Desinfektionsmittel im Raum durch mindestens einen Sensor überwacht wird.

Der Erfindung liegt die Aufgabe zugrunde, die gattungsgemäße Desinfektionseinrichtung sowie das gattungsgemäße Verfahren so auszubilden, dass die Desinfektion des Raumes innerhalb kurzer Zeit einwandfrei und ohne großen Aufwand möglich ist.

Diese Aufgabe wird bei der gattungsgemäßen Desinfektionseinrichtung erfindungsgemäß mit den kennzeichnenden Merkmalen des Anspruches 1 und beim gattungsgemäßen Verfahren erfindungsgemäß mit den kennzeichnenden Merkmalen des Anspruches 11 gelöst.

Die erfindungsgemäße Desinfektionseinrichtung ermöglicht es, den Desinfektionsvorgang vollautomatisch durchzuführen. Der Sensor überwacht während des Desinfektionsvorganges den entsprechenden Parameter und sendet entsprechende Signale an die Steuerung. Die Sensorsignale werden dazu ausgenutzt, das Schaltventil zu betätigen, mit dem das Desinfektionsmittel in den Raum ausgebracht wird. Die Steuerung sorgt dafür, dass das Desinfektionsmittel so ausgebracht wird, dass es oberhalb des vorgegebenen Mindestwertes liegt. Es ist nicht mehr erforderlich, nach der Messung des Parameters den zu desinfizierenden Raum zu betreten, um den entsprechenden Parameter auf den gewünschten Wert zu bringen.

Der Sensor ist beispielhaft ein Temperatursensor, mit dem die Raumtemperatur erfasst und überwacht wird. Da der Sensor an die Steuerung angeschlossen ist, ist sichergestellt, dass die für den Desinfektionsvorgang erforderliche Mindestraumtemperatur eingehalten wird.

Bei einer vorteilhaften Ausführungsform ist an die Steuerung wenigstens ein weiterer Sensor angeschlossen. Mit ihm kann ein weiterer Parameter im zu desinfizierenden Raum erfasst und überwacht werden. Dadurch kann der Desinfektionsvorgang optimiert werden.

Der weitere Sensor ist vorteilhaft zur Erfassung und Überwachung der relativen Feuchte im Raum vorgesehen. Insbesondere zu Beginn des Desinfektionsvorganges ist es erforderlich, dass im zu desinfizierenden Raum eine vorgegebene Mindestfeuchtigkeit vorhanden ist. Da der weitere Sensor die Feuchtigkeit erfasst, beginnt der Desinfektionsvorgang erst dann, wenn die relative Feuchte im Raum den erforderlichen Mindestwert hat.

In gleicher Weise wirkt der Temperatursensor, der insbesondere zu Beginn des Desinfektionsvorganges überprüft, ob die Raumtemperatur den vorgeschriebenen Mindestwert aufweist. Darum kann durch diesen Sensor ebenfalls sichergestellt werden, dass der Desinfektionsvorgang erst dann beginnt, wenn die erforderliche Mindesttemperatur im zu desinfizierenden Raum vorhanden ist.

Sollte die Raumtemperatur unterhalb des vorgeschriebenen Grenzwertes liegen, kann beispielsweise durch die Steuerung eine Heizeinrichtung eingeschaltet werden, die an der Desinfektionseinrichtung vorgesehen sein kann. Eine weitere Möglichkeit besteht darin, dass die Raumheizung durch Signale der Steuerung ferngesteuert eingeschaltet wird. Der Temperatursensor überwacht die Raumtemperatur. Sobald sie ihren erforderlichen Mindestwert erreicht hat, kann die Steuerung die Heizeinrichtung der Desinfektionseinrichtung oder die Raumheizung entweder drosseln oder abschalten und dann den Desinfektionsvorgang starten. Vorteilhaft ist ein dritter Sensor vorgesehen, mit dem während des Desinfektionsvorganges die Konzentration des Desinfektionsmittels im Raum erfasst und überwacht wird. Dadurch ist sichergestellt, dass sich die Konzentration des Desinfektionsmittels oberhalb eines vorgeschriebenen Mindestwertes liegt. Sollte sich die Konzentration an Desinfektionsmittel im Raum dem Grenzwert nach unten nähern, sorgt die Steuerung dafür, dass mehr Desinfektionsmittel in den Raum ausgebracht wird, um die Konzentration des Desinfektionsmittels oberhalb des Grenzwertes zu halten.

Das Desinfektionsmittel befindet sich im Behälter, der an der Desinfektionseinrichtung vorgesehen ist. Bei einer vorteilhaften Ausbildung ist für das Desinfektionsmittel wenigstens ein weiterer Behälter vorgesehen. Dadurch besteht die Möglichkeit, in den beiden Behältern unterschiedliche Konzentrationen an Desinfektionsmittel vorzuhalten. Der weitere Behälter ist ebenfalls über eine Leitung an die Austrageinheit angeschlossen. Es besteht bei einer solchen vorteilhaften Ausbildung die Möglichkeit, je nach den Konzentrationsbedingungen im Raum ein Desinfektionsmittel mit geringerer oder mit höherer Konzentration auszubringen. Dadurch kann der Desinfektionsvorgang mit nur einer minimalen Menge an Desinfektionsmittel durchgeführt werden.

In der Leitung des weiteren Behälters sitzt vorteilhaft ein Schaltventil, das an die Steuerung angeschlossen ist. Somit kann die Steuerung durch Betätigung des Schaltventiles die Zufuhr des im weiteren Behälter befindlichen Desinfektionsmittels steuern.

Bei einer bevorzugten Ausführungsform sind die Leitungen der Behälter an eine Sammelleitung angeschlossen. Dies ergibt einen konstruktiv einfachen Aufbau der Desinfektionseinrichtung. Durch Betätigen des entsprechenden Schaltventiles durch die Steuerung wird dafür gesorgt, dass nur das Desinfektionsmittel des einen Behälters zur Austrageinheit gelangt.

Die Austrageinheit weist vorteilhaft wenigstens eine Sprühdüse auf. Mit ihr kann das Desinfektionsmittel im Raum fein versprüht ausgebracht werden. Es bildet sich ein Aerosolnebel, der sich auf allen Flächen im Raum niedersetzt. So ist es beispielsweise nicht erforderlich, im Raum befindliche Vorhänge vor dem Desinfektionsvorgang zu entfernen. Der Sprühnebel sorgt dafür, dass auch die Vorhänge einwandfrei desinfiziert werden.

Besonders vorteilhaft ist es, wenn die Desinfektionseinrichtung wenigstens einen Wasseranschluss aufweist, der mit der Austrageinheit über eine Leitung verbunden ist, in der ein Schaltventil sitzt. Dadurch besteht die Möglichkeit, durch Öffnen des Schaltventils in den Raum Feuchtigkeit einzubringen, falls die relative Feuchte im Raum zu niedrig sein sollte. Der Wasseranschluss kann über eine Leitung mit einem Wasserbehälter verbunden sein, der Bestandteil der Desinfektionseinrichtung ist.

Das Schaltventil für den Wasseranschluss ist vorteilhaft ebenfalls an die Steuerung angeschlossen, so dass über die Steuerung gezielt so viel Feuchtigkeit in den Raum eingebracht werden kann, dass er die erforderliche Mindestfeuchte aufweist.

Beim erfindungsgemäßen Verfahren wird das Desinfektionsmittel gesteuert im Sprühverfahren in den Raum ausgebracht. Hierbei wird der Gehalt an Desinfektionsmittel durch wenigstens einen Sensor so überwacht, dass der Konzentrationswert des Desinfektionsmittels im Raum über einem vorgegebenem Mindestwert liegt. Dadurch ist eine vollautomatische Desinfizierung des Raumes möglich. Die Steuerung sorgt dafür, dass die Konzentration an Desinfektionsmittel im Raum nicht unterhalb des zulässigen Mindestwertes liegt.

Vorteilhaft wird das Desinfektionsmittel als Aerosol ausgesprüht. Dadurch werden alle Flächen im Raum zuverlässig durch das Desinfektionsmittel so beaufschlagt, dass sie einwandfrei desinfiziert werden. Durch die Aerosoldesinfektion werden auch Eckbereiche oder Kantenbereiche zuverlässig erfasst.

Es besteht bei diesem Verfahren sogar die Möglichkeit, zu Beginn des Desinfektionsvorganges in den Raum weitere zu desinfizierende Gegenstände zu bringen, die dann durch das Aerosol erfasst und somit desinfiziert werden.

Die Signale des Sensors werden vorteilhaft einer Steuerung zugeführt, die in Abhängigkeit von den Sensorsignalen den Austrag des Desinfektionsmittels regelt. Die Regelung führt dazu, dass das Desinfektionsmittel durch die Steuerung automatisch im erforderlichen Maße in den Raum gesprüht wird. Die Steuerung vergleicht die vom Sensor kommenden Signale mit einem vorgegebenen Wert und stellt dadurch fest, ob der Istwert dem Sollwert entspricht. Sollte dies nicht der Fall sein, sorgt die Regelung dafür, dass das Desinfektionsmittel stets auf dem gewünschten, oberhalb des vorgeschriebenen Mindestwertes liegenden Wert liegt.

Der Austrag des Desinfektionsmittels wird bei einer vorteilhaften Ausführungsform dann gestartet, wenn die Raumtemperatur und/oder die relative Feuchte im Raum oberhalb vorgegebener Mindestwerte liegen. Hierfür können entsprechende Sensoren vorgesehen werden, welche die Raumtemperatur sowie die relative Feuchte im Raum erfassen und überwachen. Zu Beginn des Desinfektionsvorganges überprüfen die Sensoren die Raumtemperatur und die Raumfeuchte. Liegen die entsprechenden Werte unterhalb der erforderlichen Mindestwerte, beginnt der Desinfektionsvorgang nicht. Die Steuerung, welcher diese Signale zugeführt werden, sorgt zunächst dafür, dass die Raumtemperatur und die relative Feuchte im Raum auf die erforderlichen Mindestwerte erhöht werden. Erst dann wird über die Steuerung ein entsprechendes Einschaltsignal erzeugt, wodurch der Desinfektionsvorgang gestartet wird.

Die Schaltventile werden bevorzugt in Abhängigkeit von den Sensorsignalen durch die Steuerung geschaltet. Dadurch ist sichergestellt, dass der Desinfektionsvorgang vollautomatisch ausgeführt werden kann. Die Steuerung wertet die zugeführten Sensorsignale aus und sorgt dafür, dass die Schaltventile so eingestellt werden, dass die Desinfektion des Raumes unter den erforderlichen Vorbedingungen einwandfrei durchgeführt wird.

Die Erfindung wird anhand eines in den Zeichnungen dargestellten Ausführungsbeispieles näher erläutert. Es zeigen
- Fig. 1: in schematischer Darstellung eine erfindungsgemäße Desinfektionseinrichtung,
- Fig. 2: ein Ablaufdiagramm für einen Desinfektionsvorgang mit Hilfe der erfindungsgemäßen Desinfektionseinrichtung,
- Fig. 3: in einem Ablaufdiagramm eine Regelung zur Durchführung des Desinfektionsvorganges,
- Fig. 4: ein beispielhaftes Applikationsprotokoll, das bei Anwendung des Desinfektionsverfahrens ausgestellt wird.

Mit der Desinfektionseinrichtung werden Räume, insbesondere Räume in Krankenhäusern, keimfrei gemacht. Hierbei wird eine Aerosoldesinfektion mit einem flüssigen Desinfektionsmittel vorgenommen. Als geeignete Desinfektionsmittel kommen beispielhaft Wasserstoffperoxid, Peressigsäure, Glutaraldehyd oder Alkohol in verschiedenen Konzentrationen und/oder Kombinationen in Betracht. Abhängig vom eingesetzten Desinfektionsmittel sind bestimmte Voraussetzungen zu erfüllen, um das gewünschte Ergebnis eines keimfreien Raumes zu erzielen.

Vorteilhaft wird Wasserstoffperoxid als Desinfektionsmittel eingesetzt. Bei diesem Desinfektionsmittel muss beispielsweise die Raumtemperatur mindestens 20° C und die relative Luftfeuchte mindestens 60% betragen. Ein Anstieg der relativen Luftfeuchte über einen Wert von 95% sollte vermieden werden, da es hierbei zu einer unkontrollierten Kondensation des Desinfektionsmittels an der Raumdecke kommen kann. Das kondensierte Desinfektionsmittel würde dann abtropfen und beispielsweise den Boden oder auch Einrichtungsgegenstände beschädigen.

Die Desinfektionseinrichtung ist so ausgebildet, dass die Desinfektion des Raumes vollautomatisch durchgeführt werden kann. Während des Desinfektionsvorganges muss keine Bedienperson den zu desinfizierenden Raum betreten. Dies ist erst notwendig, wenn der Desinfektionsvorgang abgeschlossen ist.

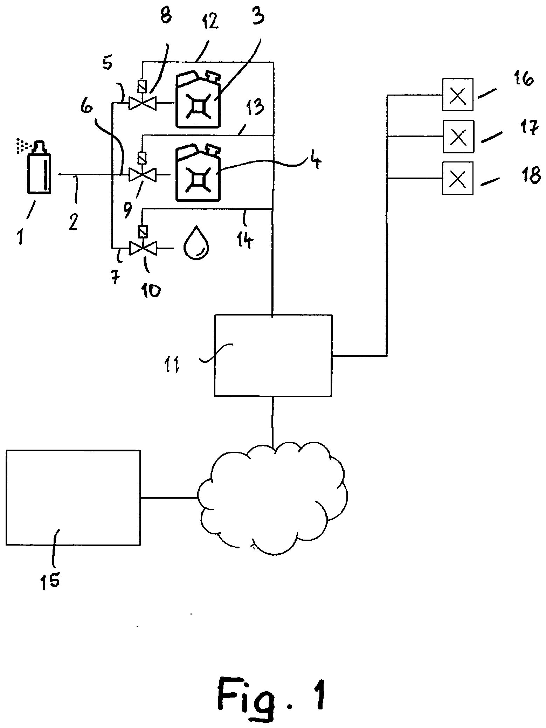

Die Desinfektionseinrichtung hat wenigstens eine Austragdüse 1, die in Fig. 1 schematisch dargestellt ist. Über sie wird das Desinfektionsmittel ausgetragen. An die Austragdüse 1 ist eine Sammelleitung 2 angeschlossen, über die das Desinfektionsmittel zugeführt wird.

Das Desinfektionsmittel ist in wenigstens einem Behälter, im Ausführungsbeispiel in zwei Behältern 3, 4 untergebracht. Beide Behälter weisen bevorzugt das Desinfektionsmittel in zwei verschiedenen Konzentrationen auf. Grundsätzlich ist es möglich, dass in den Behältern 3, 4 das Desinfektionsmittel mit jeweils gleicher Konzentration enthalten ist. Die Behälter 3, 4 bestehen aus einem gegen das Desinfektionsmittel beständigen Material, vorzugsweise aus einem geeigneten Kunststoff.

Darüber hinaus kann ein weiterer (nicht dargestellter) Behälter vorgesehen sein, der beispielsweise Wasser enthält.

Die Behälter 3, 4 sind über jeweils eine Leitung 5, 6 an die Sammelleitung 2 angeschlossen. Der (nicht dargestellte) Wasserbehälter ist über eine Leitung 7 mit der Sammelleitung 2 verbunden. In den Leitungen 5 bis 7 sitzt jeweils ein Schaltventil 8, 9, 10. Über eine Steuerung 11 werden die Ventile 8 bis 10 über entsprechende Steuerleitungen 12 bis 14 angesteuert.

Die Steuerung 11 kann über einen Computer 15, ein Tablet oder dergleichen kabelgebunden oder kabellos ferngesteuert werden. Die Bedienperson muss darum den zu desinfizierenden Raum nicht betreten, um Einstellungen an der Steuerung 11 vorzunehmen. In die Steuerung 11 werden vom Anwender über den Computer 15 und dergleichen die für den Desinfektionsvorgang erforderlichen Parameter eingegeben. Im Ausführungsbeispiel sind dies die Raumtemperatur, die relative Luftfeuchtigkeit im zu desinfizierenden Raum sowie die Konzentration des Desinfektionsmittels im Raum. Diese Parameter werden in der Regel durch Tests ermittelt, die von Fachleuten durchgeführt werden. Es besteht auch die Möglichkeit, für gängige Desinfektionsmittel die einzuhaltenden Parameter in der Steuerung abzuspeichern. Der Anwender muss dann die Parameter nicht selbst eingeben, sondern nur das einzusetzende Desinfektionsmittel auswählen.

An die Steuerung 11 sind Sensoren 16 bis 18 angeschlossen, mit denen für den Desinfiziervorgang notwendige Parameter im zu desinfizierenden Raum erfasst werden. Mit dem Sensor 16 wird beispielsweise die Raumtemperatur, mit dem Sensor 17 der Raumfeuchtegehalt in Prozent und mit dem Sensor 18 die Konzentration des Desinfektionsmittels im Raum erfasst. Die Steuerung 11 wertet die Sensorsignale aus und steuert die Schaltventile 8 bis 10 derart, dass die gewünschten und für den Desinfektionsvorgang erforderlichen Bedingungen eingehalten werden. Wie in Bezug auf Wasserstoffperoxid beispielhaft erläutert, muss die Raumtemperatur wenigstens 20° C und die relative Luftfeuchte mindestens 60% betragen. Dies lässt sich mit den Sensoren 16 und 17 sehr einfach überwachen. Solange diese Werte nicht erreicht sind, beginnt die Desinfektionseinrichtung den Desinfiziervorgang nicht.

Zum Ausbringen des Desinfektionsmittels ist die Einrichtung mit einem Druckkompressor versehen. An ihn sind die Behälter 3, 4 in bekannter Weise angeschlossen, so dass bei Einschalten des Kompressors das jeweilige Medium ausgetragen werden kann. Das Medium wird bei Einschalten des Kompressors angesaugt und in die jeweiligen Leitungen 5 bis 7 gefördert. Der Kompressor ist ebenfalls an die Steuerung 11 angeschlossen, so dass mit ihr die Desinfektionseinrichtung ferngesteuert ein- bzw. ausgeschaltet werden kann.

Mit der Desinfektionseinrichtung kann der jeweilige Raum entsprechend den erforderlichen Vorschriften desinfiziert werden, ohne dass es notwendig ist, den zu desinfizierenden Raum zu betreten. Die einzelnen Komponenten der Desinfektionseinrichtung befinden sich vorteilhaft auf einem verfahrbaren Wagen, der sich leicht in den zu desinfizierenden Raum bewegen lässt. Da das Desinfektionsmittel vernebelt wird, werden alle Flächen im Raum zuverlässig vom Desinfektionsmittel erfasst. Die Vernebelung des Desinfektionsmittels gewährleistet, dass das Desinfektionsmittel in jeden Bereich des Raumes gelangt. So ist es beispielsweise auch nicht notwendig, die Vorhänge im Raum zu entfernen. Das vernebelte Desinfektionsmittel setzt sich auf den Vorhängen ab, so dass auch diese ohne zusätzliche Reinigungsarbeit desinfiziert werden.

Durch die Vernebelung ist es auch möglich, Innenräume von Schränken, Kästen und dergleichen zu desinfizieren. Es ist lediglich notwendig, die jeweiligen Türen zu öffnen, damit das Desinfektionsmittel in das Innere gelangen kann.

Es hat sich als vorteilhaft erwiesen, für das ausgesprühte Desinfektionsmittel ein Tropfenspektrum zu wählen, bei dem die Tropfendurchmesser in einem Bereich von etwa 6 bis etwa 80 µm liegen. Ein besonders vorteilhafter Desinfektionsvorgang ergibt sich, wenn etwa 90 % der Tropfen des Desinfektionsmittels in einem Durchmesserbereich zwischen etwa 9 und etwa 40 µm oder 95 % in einem Durchmesserbereich zwischen etwa 7 µm und etwa 45 µm liegen. Bei einem derartigen Tropenspektrum werden die Flächen im Raum zuverlässig mit dem Desinfektionsmittel benetzt.

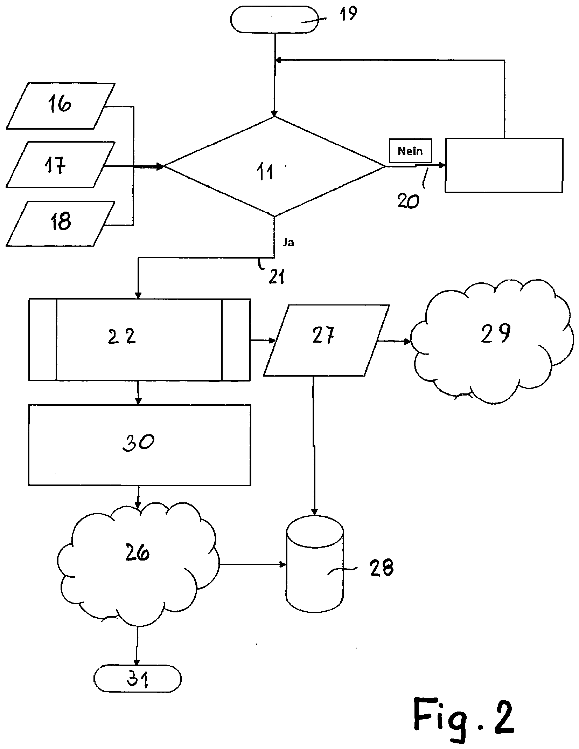

Anhand von Fig. 2 wird beispielhaft ein Desinfektionsvorgang beschrieben. Die Desinfektionseinrichtung wird im zu desinfizierenden Raum untergebracht. Während des Desinfektionsvorganges befinden sich keine Personen im Raum. Die Desinfektionseinrichtung kann mittels der Fernsteuerung 15 von außen betätigt werden. Zunächst wird über diese Fernbedienung 15 das Startsignal 19 erzeugt und damit die Einrichtung eingeschaltet. Die Schaltventile 8 bis 10 sind beim Einschaltvorgang geschlossen, so dass kein Medium zur Austragdüse 1 gelangen kann. Die Sensoren 16 bis 18 werden eingeschaltet. Im Beispielsfall misst der Sensor 16 die Raumtemperatur und der Sensor 17 die relative Feuchte im Raum. Die Sensorsignale werden der Steuerung 11 zugeführt, die überprüft, ob die Mindesttemperatur sowie der Mindestwert der relativen Luftfeuchte vorhanden sind. Ist dies nicht der Fall, wird ein entsprechendes Signal 20 von der Steuerung 11 ausgegeben. Nunmehr können Vorkehrungen getroffen werden, um die Raumtemperatur auf den Mindesttemperaturwert und den Feuchtegehalt auf den Mindestwert zu bringen. Sobald die entsprechenden Sensoren der Steuerung 11 signalisieren, dass die Mindestwerte erreicht sind, gibt die Steuerung 11 ein Startsignal 21, so dass der Desinfiziervorgang gestartet wird (22).

Während des Desinfiziervorganges überwachen die Sensoren 16 und 17, ob die Raumtemperatur und die relative Feuchte oberhalb der erforderlichen Mindestwerte liegen. Der Sensor 18 dient beispielhaft dazu, die Konzentration des Desinfektionsmittels im Raum zu überwachen. Bei Verwendung von Wasserstoffperoxid als Desinfektionsmittel muss die Konzentration an Wasserstoffperoxid oberhalb von 100 ppm pro Stunde liegen. Sollte dieser Konzentrationswert absinken, wird durch die Steuerung 11 das Ventil 8 und/oder 9 weiter geöffnet, so dass mehr Desinfektionsmittel aus dem Behälter 3 bzw. 4 zugeführt wird. Sobald der Sensor 18 der Steuerung 11 meldet, dass der vorgegebene Konzentrationswert erreicht ist, kann die Steuerung 11 das entsprechende Ventil 8, 9 wieder so einstellen, dass entsprechend weniger Desinfektionsmittel ausgetragen wird.

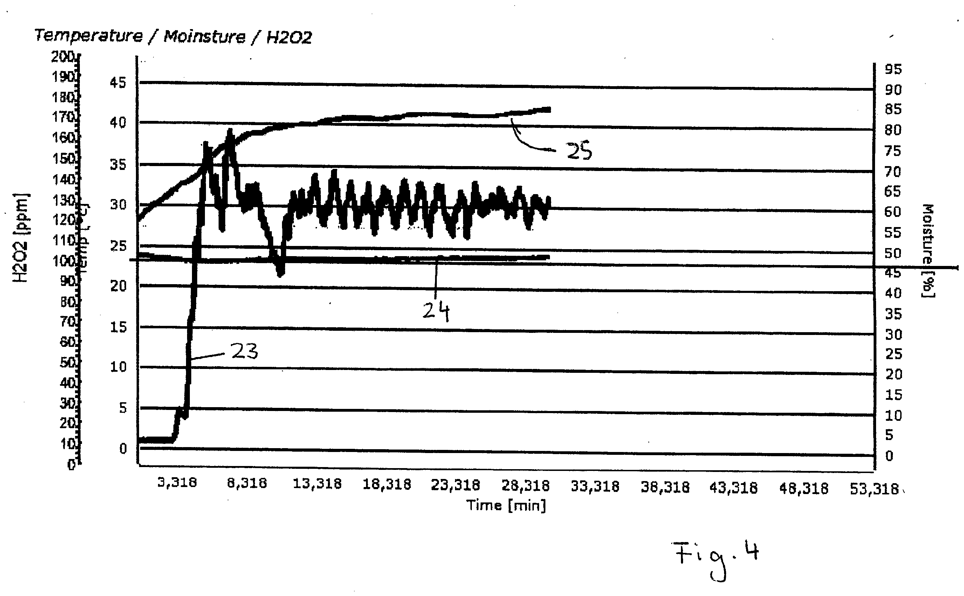

Auf die beschriebene Weise wird der Desinfektionsvorgang geregelt durchgeführt, so dass der Verbrauch an Desinfektionsmittel minimiert werden kann. Je nach eingesetztem Desinfektionsmittel können die durch die Sensoren 16 bis 18 zu erfassenden Parameter unterschiedlich sein. Die für das jeweils eingesetzte Desinfektionsmittel gültigen Parameter können in der Steuerung dauerhaft abgelegt oder von außen eingespielt werden. Der Desinfektionsvorgang wird in vorteilhafter Weise protokolliert, so dass der Desinfiziervorgang jederzeit nachverfolgt werden kann. Fig. 4 zeigt beispielhaft ein solches Protokoll. In ihm sind die Konzentration des Desinfektionsmittels (Wasserstoffperoxid), die Raumtemperatur und der Feuchtegehalt im Raum gegen die Zeit aufgetragen. Die Kurve 23 kennzeichnet die Konzentration an Desinfektionsmittel, die Kurve 24 die Raumtemperatur, die über die Dauer des Desinfiziervorganges oberhalb der Mindesttemperatur von 20° C liegt. Die Kurve 25 zeigt, dass die relative Luftfeuchte oberhalb von 60% liegt. Die Konzentrationskurve 23 verdeutlicht, dass der bei Verwendung von Wasserstoffperoxid als Desinfektionsmittel erforderliche Konzentrationswert während der Dauer des Desinfiziervorganges oberhalb von 100 ppm liegt. Bei Verwendung eines anderen Desinfektionsmittels können die Parameter Temperatur, relative Feuchte und Konzentration bei anderen Werten liegen. Dementsprechend werden diese Werte in die Steuerung 11 eingegeben, damit sie die erforderliche Regelung des Desinfizierprozesses durchführen kann.

Das Protokoll 26 (Fig. 2) wird gespeichert und/oder versandt.

Die von den Sensoren 16 bis 18 erfassten und der Steuerung 11 zugeführten Sensorwerte werden im Schritt 27 geschrieben. Hierbei können die Sensorwerte einem Datenspeicher 28 zugeführt werden, in dem die Sensorwerte 27 gespeichert werden. Auch das Protokoll 26 kann im Datenspeicher 28 abgelegt werden.

Die Sensorwerte können darüber hinaus auch online zur Verfügung gestellt werden (Schritt 29). Für den Benutzer der Einrichtung besteht dadurch die Möglichkeit, bereits während des Desinfizierprozesses die kritischen Sensorwerte zu überwachen und im Gefahrenfall einzugreifen.

Vorteilhaft ist es, wenn nach dem Desinfiziervorgang im Schritt 22 anschließend ein Reinigungsvorgang 30 durchgeführt wird. Hierfür wird das Wasser eingesetzt, das durch die Leitungen gespült und über die Austragdüse 1 ausgesprüht wird. Dieses Wasser ist für den desinfizierten Raum nicht kritisch.

Nach Abschluss des Desinfiziervorganges wird über die Steuerung 11 im Schritt 31 die Einrichtung abgeschaltet.

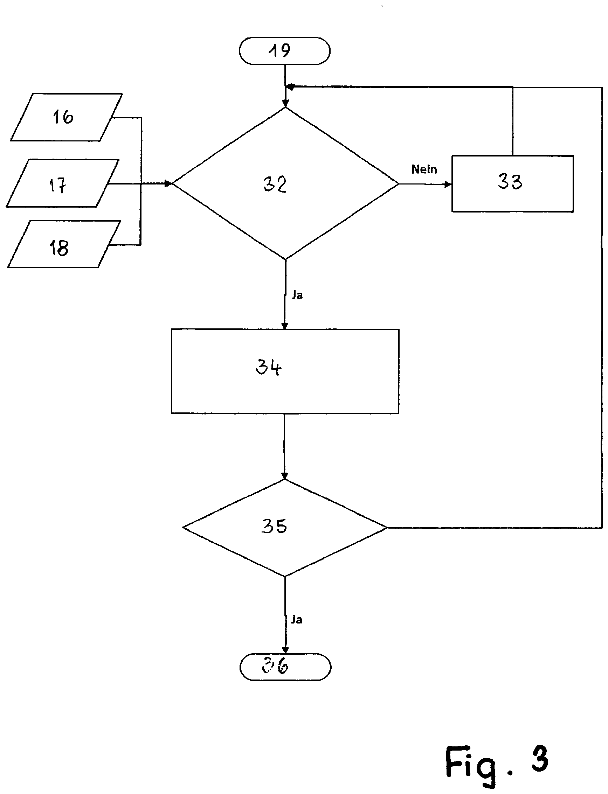

Anhand von Fig. 3 wird die zuvor kurz erläuterte Regelung des Desinfizierprozesses näher erläutert.

Zum Startzeitpunkt 19 wird im Schritt 32 überprüft, ob die Konzentration des eingesetzten Desinfektionsmittels ausreichend ist und ob die für den Desinfektionsvorgang erforderlichen Vorbedingungen (Raumtemperatur, relative Feuchte) erfüllt sind. Die entsprechenden Werte werden in der beschriebenen Weise über die Sensoren 16, 17 der Steuerung 11 mitgeteilt. Sind die Vorbedingungen zur Einleitung des Desinfiziervorganges nicht gegeben, wird der mit dem Startsignal 19 gestartete Timer im Schritt 33 gestoppt, so dass die Voraussetzungen im zu desinfizierenden Raum hergestellt werden können. Die Überwachung der Raumtemperatur sowie der relativen Feuchte im Raum wird weiterhin durch die Sensoren 16, 17 überwacht. Sobald die Raumtemperatur sowie die relative Feuchte über den erforderlichen Mindestwerten liegen, startet die Steuerung 11, welche die entsprechenden Sensorsignale erhält, den Desinfiziervorgang erneut (Schritt 34). Der Timer wird erneut eingeschaltet. Dabei kann der Timer die Zeit weiter zählen oder die Timerzeit wird auf null zurückgesetzt und erneut gestartet.

Während des Desinfektionsvorganges regelt die Steuerung 11 in der beschriebenen Weise das Ausbringen des Desinfektionsmittels. Der Sensor 18 überwacht hierbei die Konzentration des Desinfektionsmittels im Raum, damit die Mindestkonzentrationswerte nicht unterschritten werden. Bei Verwendung von Wasserstoffperoxid als Desinfektionsmittel darf die Konzentration über einen Zeitraum von einer Stunde nicht geringer als 100 ppm sein.

Im Schritt 35 überprüft die Steuerung 11, ob die eingestellte Timerzeit abgelaufen ist. Ist dies der Fall, sendet die Steuerung 11 ein Abschaltsignal 36, wodurch die Desinfiziereinrichtung abgeschaltet wird.

Im Schritt 35 kann die Steuerung auch überprüfen, ob der vorgeschriebene Konzentrationswert des Desinfektionsmittels innerhalb der Dauer von einer Stunde oberhalb des vorgeschriebenen Mindestwertes lag. Sollte dies nicht der Fall sein, verlängert die Steuerung 11 die Timerlaufzeit um die fehlende Restlaufzeit. Erst dann wird das Abschaltsignal erzeugt.

Die beschriebene Desinfektionseinrichtung arbeitet zuverlässig und vollautomatisch. Zur Bedienung der Desinfektionseinrichtung ist kein geschultes Personal notwendig. Die Regelung sorgt dafür, dass das Desinfektionsmittel nur in dem Maße ausgetragen wird, wie es zur sicheren Desinfizierung des Raumes erforderlich ist. Der Verbrauch an Desinfektionsmittel ist darum gering. Auch die Dauer des Desinfektionsvorganges kann aufgrund der beschriebenen Regelung minimiert werden. Darum steht der zu desinfizierende Raum auch wieder frühzeitig zur Verfügung, was insbesondere in Krankenhäusern von großem Vorteil ist.

Da das Desinfektionsmittel in den Raum gesprüht wird, werden alle Flächen im Raum desinfiziert. Es besteht sogar die Möglichkeit, vor Beginn des Desinfektionsprozesses in den zu desinfizierenden Raum weitere Gegenstände, Geräte und dergleichen zu bringen, die beim anschließenden Desinfiziervorgang ebenfalls desinfiziert werden können.

Da die Desinfiziereinrichtung an den Wasseranschluss angeschlossen werden kann bzw. den Wasserbehälter aufweist, besteht die Möglichkeit, die Raumfeuchte auf den erforderlichen Mindestwert zur erhöhen, falls dies erforderlich sein sollte. In diesem Falle werden vor Beginn des Desinfiziervorganges die Schaltventile 8, 9 geschlossen gehalten und das Schaltventil 10 geöffnet. Dieser Befeuchtungsvorgang erfolgt ebenfalls in vorteilhafter Weise automatisch. Der Sensor 17 erfasst die Raumfeuchte und kann somit während des Einsprühens von Wasser den Feuchtegehalt im zu desinfizierenden Raum überprüfen. Die vom Sensor 17 an die Steuerung 11 gelieferten Signale werden ausgewertet, so dass der Desinfiziervorgang erst dann stattfindet, wenn die Voraussetzungen zur Durchführung des Desinfiziervorganges gegeben sind, wie zuvor erläutert worden ist.

## Patentansprüche

1. Desinfektionseinrichtung zum Desinfizieren von Räumen, mit wenigstens einem Behälter (3, 4) für ein Desinfektionsmittel, an den über eine Leitung (5, 6), in der ein Schaltventil (8, 9) sitzt, wenigstens eine Austrageinheit (1) angeschlossen ist, wobei das Schaltventil (8, 9) an eine Steuerung (11) angeschlossen ist, an die wenigstens ein Sensor (16 bis 18) angeschlossen ist, und mit einem Kompressor,
**dadurch gekennzeichnet, dass** die Leitung (5, 6) an eine Sammelleitung (2) anschließt, die an die Austrageinheit (1) angeschlossen ist, dass an die Sammelleitung (2) wenigstens ein Wasseranschluss über eine Leitung (7) angeschlossen ist, in der ein an die Steuerung (11) angeschlossenes Schaltventil (10) sitzt, dass der Sensor (16 bis 18) einen für den Desinfektionsvorgang kritischen Parameter im zu desinfizierenden Raum überwacht, dass die Austrageinheit (1) wenigstens eine Sprühdüse aufweist, und dass der Kompressor an die Steuerung (11) angeschlossen und ein Druckkompressor ist, an den der Behälter (3, 4) für das Desinfektionsmittel angeschlossen ist, aus dem bei Einschalten des Kompressors das Desinfektionsmittel angesaugt und in die Leitung (5, 6) des Behälters (3, 4) und in die Leitung (7) des Wasseranschlusses gefördert wird.

2. Einrichtung nach Anspruch 1,
**dadurch gekennzeichnet, dass** der Sensor (16) ein Temperatursensor ist.

3. Einrichtung nach Anspruch 1 oder 2,
**dadurch gekennzeichnet, dass** wenigstens ein weiterer Sensor (17, 18) an die Steuerung (11) angeschlossen ist, der vorteilhaft die relative Feuchtigkeit im Raum erfasst.

4. Einrichtung nach Anspruch 3
**dadurch gekennzeichnet, dass** ein weiterer Sensor (18) die Konzentration an Desinfektionsmitteln im Raum erfasst.

5. Einrichtung nach einem der Ansprüche 1 bis 4,
**dadurch gekennzeichnet, dass** wenigstens ein weiterer Behälter (3, 4) vorgesehen ist, der über eine Leitung (5, 6) an die Austrageinheit (1) angeschlossen ist.

6. Einrichtung nach Anspruch 5,
**dadurch gekennzeichnet, dass** in der Leitung (5, 6) des weiteren Behälters (3, 4) ein Schaltventil (8, 9) sitzt, das an die Steuerung (11) angeschlossen ist.

7. Verfahren zum Desinfizieren von Räumen unter Verwendung einer Desinfektionseinrichtung nach einem der Ansprüche 1 bis 6, bei dem ein Desinfektionsmittel in den zu desinfizierenden Raum gesteuert im Sprühverfahren eingebracht wird,
**dadurch gekennzeichnet, dass** der Gehalt an Desinfektionsmittel im Raum durch wenigstens einen Sensor (16 bis 18) so überwacht wird, dass der Konzentrationswert des Desinfektionsmittels im Raum über einem vorgegebenen Mindestwert liegt, wobei das Desinfektionsmittel einen Tropfendurchmesser in einem Bereich von etwa 6µm bis etwa 80 µm aufweist, wobei etwa 90% der Tropfen des Desinfektionsmittels in einem Durchmesserbereich zwischen etwa 9 und etwa 50µm oder 85% in einem Druchmesserbereich zwischen etwa 7µm und etwa 45µm liegen.

8. Verfahren nach Anspruch 7,
**dadurch gekennzeichnet, dass** das Desinfektionsmittel als Aerosol ausgesprüht wird.

9. Verfahren nach Anspruch 7 oder 8,
**dadurch gekennzeichnet, dass** die Signale des Sensors (16 bis 18) einer Steuerung (11) zugeführt werden, die in Abhängigkeit von den Sensorsignalen den Austrag des Desinfektionsmittels regelt.

10. Verfahren nach einem der Ansprüche 7 bis 9,
**dadurch gekennzeichnet, dass** der Austrag des Desinfektionsmittels gestartet wird, wenn die Raumtemperatur und/oder die relative Feuchte im Raum oberhalb vorgegebener Mindestwerte liegen.

11. Verfahren nach einem der Ansprüche 7 bis 10,
**dadurch gekennzeichnet, dass** die Schaltventile (8 bis 10) in Abhängigkeit von den Sensorsignalen durch die Steuerung (11) geschaltet werden.

## Claims

1. A disinfection device for disinfecting rooms with at least one container (3, 4) for a disinfectant, to which at least one discharge unit (1) is connected via a line (5, 6) containing a switching valve (8, 9), wherein the switching valve (8, 9) is connected to a control (11), to which at least one sensor (16 to 18) is connected, and with a compressor,
**characterized in that** the line (5, 6) is connected to a collecting line (2) that is connected to the discharge unit (1), **in that** at least one water connection is connected to the collecting line (2) via a line (7) containing a switching valve (10) connected to the control (11), **in that** the sensor (16 to 18) monitors a critical parameter for the disinfection process in the room to be disinfected, **in that** the discharge unit (1) has at least one spray nozzle, and **in that** the compressor is connected to the control (11) and is a pressure compressor, to which the container (3, 4) for the disinfectant is connected, from which the disinfectant is drawn when the compressor is switched on and conveyed into the line (5, 6) of the container (3, 4) and into the line (7) of the water connection.

2. The device according to claim 1,
**characterized in that** the sensor (16) is a temperature sensor.

3. The device according to claim 1 or 2,
**characterized in that** at least one additional sensor (17, 18), which advantageously measures the relative humidity in the room, is connected to the control (11).

4. The device according to claim 3,
**characterized in that** an additional sensor (18) measures the concentration of disinfectants in the room.

5. The device according to one of claims 1 to 4,
**characterized in that** at least one additional container (3, 4) is provided and connected to the discharge unit (1) via a line (5, 6).

6. The device according to claim 5,
**characterized in that** the line (5, 6) of the additional container (3, 4) contains a switching valve (8, 9) that is connected to the control (11).

7. A method for disinfecting rooms by using a disinfection device according to one of claims 1 to 6, wherein a disinfectant is introduced into the room to be disinfected in a controlled manner in a spraying process,
**characterized in that** the content of disinfectant in the room is monitored by at least one sensor (16 to 18) in such a way that the concentration value of the disinfectant in the room lies above a predefined minimum value, wherein the disinfectant has a droplet diameter in a range between approximately 6 µm and approximately 80 µm, and wherein approximately 90 % of the droplets of the disinfectant lie in a diameter range between approximately 9 and approximately 50 µm or 85 % of the droplets lie in a diameter range between approximately 7 µm and approximately 45 µm.

8. The method according to claim 7,
**characterized in that** the disinfectant is sprayed out in the form of an aerosol.

9. The method according to claim 7 or 8,
**characterized in that** the signals of the sensor (16 to 18) are fed to a control (11), which controls the discharge of the disinfectant in dependence on the sensor signals.

10. The method according to one of claims 7 to 9,
**characterized in that** the discharge of the disinfectant is started when the room temperature and/or the relative humidity in the room lie above predefined minimum values.

11. The method according to one of claims 7 to 10,
**characterized in that** the switching valves (8 to 10) are switched the control (11) in dependence on the sensor signals.

## Revendications

1. Dispositif de désinfection, destiné à désinfecter des locaux, pourvu d'au moins un réservoir (3, 4) pour un agent désinfectant, sur lequel se raccorde par l'intermédiaire d'une conduite (5, 6), dans laquelle est logée une soupape de commutation (8, 9) au moins une unité d'évacuation (1), la soupape de commutation (8, 9) étant raccordée sur un système de commande (11), sur lequel est raccordé au moins un capteur (16 à 18) et pourvu d'un compresseur,
**caractérisé en ce que** la conduite (5, 6) se raccorde sur une conduite collective (2) qui est raccordée sur l'unité d'évacuation (1), **en ce que** sur la conduite collective (2), au moins un branchement d'eau est raccordé par l'intermédiaire d'une conduite (7), dans laquelle est logée une soupape de commutation (10) raccordée au système de commande (11), **en ce que** le capteur (16 à 18) supervise dans le local à désinfecter un paramètre critique pour le processus de désinfection, **en ce que** l'unité d'évacuation (1) comporte au moins une buse de pulvérisation et **en ce que** le compresseur est raccordé sur le système de commande (11) et est un compresseur à pression sur lequel est raccordé le réservoir (3, 4) pour l'agent désinfectant, à partir duquel lors de la mise en route du compresseur l'agent désinfectant est aspiré et est transporté dans la conduite (5, 6) du réservoir (3, 4) et dans la conduite (7) du branchement d'eau.

2. Dispositif selon la revendication 1,
**caractérisé en ce que** le capteur (16) est un capteur de température.

3. Dispositif selon la revendication 1 ou 2,
**caractérisé en ce qu'**au moins un capteur (17, 18) supplémentaire qui détecte avantageusement l'humidité relative dans le local est raccordé sur le système de commande (11).

4. Dispositif selon la revendication 3,
**caractérisé en ce qu'**un capteur (18) supplémentaire détecte la concentration de l'agent désinfectant dans le local.

5. Dispositif selon l'une quelconque des revendications 1 à 4,
**caractérisé en ce qu'**il est prévu au moins un réservoir (3, 4) supplémentaire, qui est raccordé par l'intermédiaire d'une conduite (5, 6) sur l'unité d'évacuation (1).

6. Dispositif selon la revendication 5,
**caractérisé en ce que** dans la conduite (5, 6) du réservoir (3, 4) supplémentaire est logée une soupape de commutation (8, 9) qui est raccordée sur le système de commande (11).

7. Procédé, destiné à désinfecter des locaux en utilisant un dispositif de désinfection selon l'une quelconque des revendications 1 à 6, lors duquel l'on introduit de manière contrôlée par procédé de pulvérisation un agent désinfectant dans le local à désinfecter,
**caractérisé en ce que** la teneur en agent désinfectant dans le local est supervisée par au moins un capteur (16 à 18) de telle sorte, que la valeur de concentration de l'agent désinfectant dans le local dépasse une valeur minimale prédéfinie, l'agent désinfectant présentant un diamètre des gouttelettes dans un ordre d'environ 6 µm à environ 80 µm, approximativement 90 % des gouttelettes de l'agent désinfectant se situant dans un diamètre de l'ordre compris entre approximativement 9 et approximativement 50 µm ou 85 % se situant dans un diamètre d'un ordre compris entre approximativement 7 µm et approximativement 45 µm.

8. Procédé selon la revendication 7,
**caractérisé en ce que** l'agent désinfectant est pulvérisé sous la forme d'un aérosol.

9. Procédé selon la revendication 7 ou 8.
**caractérisé en ce que** les signaux du capteur (16 à 18) sont amenés vers un système de commande (11), qui en fonction des signaux du capteur régule l'évacuation de l'agent désinfectant.

10. Procédé selon l'une quelconque des revendications 7 à 9,
**caractérisé en ce que** l'évacuation de l'agent désinfectant est démarrée lorsque la température ambiante et / ou l'humidité relative dans la pièce sont supérieures à des valeurs minimales prédéfinies.

11. Procédé selon l'une quelconque des revendications 7 à 10,
**caractérisé en ce que** les soupapes de commutation (8 à 10) sont commutées par le système de commande (11) en fonction des signaux des capteurs.
